Europäisches Patentamt

European Patent Office

Office européen des brevets

(11) Veröffentlichungsnummer: **0 146 113**
**A2**

## (12) EUROPÄISCHE PATENTANMELDUNG

(21) Anmeldenummer: 84115260.6

(22) Anmeldetag: 12.12.84

(51) Int. Cl.⁴: **A 61 K 37/40**, A 61 K 37/02,
C 07 K 5/00, C 07 K 7/24

(30) Priorität: 15.12.83 DE 3345397
15.12.83 DE 3345358
29.06.84 DE 3424009

(43) Veröffentlichungstag der Anmeldung: 26.06.85
Patentblatt 85/26

(84) Benannte Vertragsstaaten: AT BE CH DE FR GB IT LI LU
NL SE

(71) Anmelder: Aderhold, Dieter, Am Nocken 47,
D-5883 Kierspe (DE)

(72) Erfinder: Aderhold, Dieter, Am Nocken 47,
D-5883 Kierspe (DE)

(74) Vertreter: Dipl.-Ing. H. Hauck Dipl.-Phys. W. Schmitz
Dipl.-Ing. E. Graalfs Dipl.-Ing. W. Wehnert Dr.-Ing. W.
Döring, Neuer Wall 41, D-2000 Hamburg 36 (DE)

(54) **Wirkstoff und therapeutisches Mittel zur Behandlung von Fehlfunktionen des Diencephalon, von Nervenerkrankungen und Erkrankungen der Haut.**

(57) Es werden ein Wirkstoff und ein diesen Wirkstoff enthaltendes therapeutisches Mittel zur Therapie von Fehlfunktionen des menschlichen Diencephalon und/oder der Hypophyse, insbesondere zur Behandlung der Multiplen Sklerose, zur Therapie von Neuralgien, Neuritiden, rheumatischen Erkrankungen und zur Therapie von Hautkrankheiten beschrieben. Der Wirkstoff besteht aus $\alpha$-MSH, $\beta$-MSH und/oder $\gamma$-MSH sowie Peptid-Teilstücken dieser Substanzen. Des weiteren werden ein Wirkstoff und ein diesen Wirkstoff enthaltendes therapeutisches Mittel zur Behandlung von Zuständen infolge überhöhten Melanotropinspiegels offenbart. Dieser Wirkstoff besteht aus einem Polypeptid mit der Aminosäuresequenz Pro-Leu-Gly-$NH_2$ bzw. Teilstücken davon. Ferner werden Kombinationspräparate aus diesen Wirkstoffen vorgeschlagen.

ACTORUM AG

## Wirkstoff und therapeutisches Mittel zur Behandlung von Fehlfunktionen des Diencephalon, von Nervener- krankungen und Erkrankungen der Haut

Die vorliegende Erfindung betrifft einen Wirkstoff, bzw. ein therapeutisches Mittel zur Behandlung von Fehlfunktionen des menschlichen Diencephalons und/oder der Hypophyse und dadurch bedingter Störungen, beispielsweise des Blutdrucks, der Blasenfunktion, des hormonalen Gleichgewichts, des Stoffwechsels, der Keimdrüsenfunktionen, des Wachstums, der Wärmeregulation, des Wasserhaushalts, des Nervensystems und des psychischen Gleichgewichts, insbesondere zur Behandlung der Multiplen Sklerose.

Es ist bekannt, daß sich im Diencephalon, insbesondere im Hypothalamus, und der Hypophyse dem vegetativen Nervensystem übergeordnete Zentren befinden, welche die wichtigen Regulationsvorgänge des Organismus zusammenfassendleiten. Beispielsweise dienen diese vegetativen Zentren zur Steuerung aller wichtigen Stoffwechselvor- gänge, beispielsweise des Wärmehaushaltes, Wasserhaus- haltes, Kohlenhydratstoffwechsels, Zuckerhaushaltes, Fettumsatzes, Eiweißstoffwechsels, Grundumsatzes. Es wird angenommen, daß die eingangs genannten Störungen auf eine Hypofunktion des menschlichen Diencephalons, insbesondere des Hypothalamus, und/oder der Hypophyse zurückgehen. Auch in bezug auf die Multiple Sklerose, bei der es sich um eine der häufigsten Nervenkrankheiten mit bisher unbekannter Äthiologie handelt, wird angenommen, daß zumindest bei einem Teil der an Multipler Sklerose Erkrankten die Hypofunktion des Hypothalamus oder der Hypophyse für die Entstehung dieser Krankheit verantwortlich ist.

- 2 -

Der Erfindung liegt die Aufgabe zugrunde, einen Wirkstoff bzw. ein therapeutisches Mittel zur Behandlung von Fehlfunktionen des menschlichen Diencephalon und/oder der Hypophyse, insbesondere zur Behandlung der Multiplen Sklerose, zur Verfügung zu stellen.

Diese Aufgabe wird erfindungsgemäß durch einen Wirkstoff bzw. ein therapeutisches Mittel gelöst, das dadurch gekennzeichnet ist, daß es aus einem Gehalt von $\alpha$ -Melanotropin ( $\alpha$ -MSH), $\beta$ -Melanotropin ( $\beta$ -MSH) und/oder $\mu$ -Melanotropin ( $\mu$ -MSH) oder Peptid-Teilstücken dieser Substanzen sowie ggf. üblichen Träger- und Hilfsstoffen besteht.

Als Melanotropin bezeichnet man im Hypophysenzwischenlappen gebildete Peptidhormone, deren Sekretion durch den Hypothalamus-Faktor gehemmt wird. Bei $\alpha$ -Melanotropin ( $\alpha$ -MSH) handelt es sich um ein lineares Polypeptid mit dreizehn Aminosäuren, während es sich bei $\beta$ -MSH (Mensch) um ein Polypeptid mit achtzehn Aminosäuren handelt. Als $\mu$ -Melanotropin ( $\mu$ -MSH) wird ein Polypeptid mit zwölf Aminosäuren bezeichnet. Die vorstehend genannten Substanzen sind bereits isoliert bzw. synthetisch hergestellt worden und bekannt. Sie wurden bisher, soweit bekannt, noch nicht als therapeutisches Mittel eingesetzt.

Es wurde nunmehr gefunden, daß sich diese Substanzen in ausgezeichneter Weise zur Behandlung von Fehlfunktionen des menschlichen Diencephalon und/oder der Hypophyse und dadurch bedingter Störungen, insbesondere zur Behandlung der Multiplen Sklerose, verwenden lassen. Die Substanzen können dabei sowohl in reiner Form als auch in Verbindung mit üblichen Träger- und Hilfsstoffen appliziert werden, wobei grundsätzlich sämtliche möglichen Applikationsformen geeignet sind.

Da diese Substanzen außerordentlich empfindlich sind und sehr rasch ihre Wirksamkeit verlieren, empfiehlt es sich, die Substanzen zusammen mit einer Depotsubstanz zu applizieren. Als geeignete Depotsubstanz könnte beispielsweise das für das Hormon ACTH verwendete Depotmaterial Anwendung finden.

Melanotropin wirkt als $\alpha$-MSH praktisch in gleicher Weise wie $\beta$-MSH, was darauf zurückzuführen ist, daß beide Stoffe eine identische Sequenz von sieben Aminosäuren haben, nämlich Met-Glu-His-Phe-Arg-Trp-Gly. Erfindungsgemäß kann daher auch das Polypeptid der entsprechenden Aminosäuresequenzen selbst eingesetzt werden.

Die besten Ergebnisse wurden mit dem Tetrapeptid His-Phe-Arg-Trp erzielt, das im $\alpha$-, $\beta$- und $\gamma$-Melanotropin vorkommt. Dieses Tetrapeptid His-Phe-Arg-Trp wird als eigentlich biologisch aktiver Teil des Melanotropins angesehen.

Auch weitere Polypeptide mit der Aminosäurensequenz His-Phe-Arg-Trp, wie beispielsweise $\gamma$-MSH (3-8), haben zu guten Ergebnissen geführt. Es wird angenommen, daß die bei Multipler Sklerose (MS) auftretenden Schädigungen des Nervensystems durch die Zuführung des Hyphophysenhormons Melanotropin günstig beeinflußt werden können.

Die vorliegende Erfindung betrifft ferner einen Wirkstoff zur Therapie von Neuralgien, Neuritiden und rheumatischen Erkrankungen, Folgezuständen nach und infolge von Polioinfektionen, der Lateralsklerose sowie der Gicht. Die Erfindung bezieht sich ebenfalls auf ein therapeutisches Mittel, das einen derartigen Wirkstoff enthält.

Es ist ein Mittel zur Behandlung von rheumatischen Krankheitsbildern bekannt, bei dem es sich um ein synthetisches ACTH-Präparat handelt. Das Präparat enthält ein offenkettiges Polypeptid, das der Sequenz der ersten 24 Aminosäuren des natürlichen ACTH-Moleküls (Corticotrophin)entspricht und alle pharmakologischen Eigenschaften des körpereigenen Corticotrophins besitzt. Es ist bekannt, daß ACTH das Wachstum der Nebenrinden und die Bildung und Freisetzung von Steroid-Hormonen durch die Nebennierenrinde stimuliert, insbesondere die Bildung von Cortisol und seinen Vorstufen in der Nebennierenrinde steigert. Die therapeutischen Wirkungen des vorstehend genannten synthetischen ACTH-Präparates werden im wesentlichen auf diese Effekte zurückgeführt.

Erfindungsgemäß wird die Anwendung von $\alpha$-Melanotropin ( $\alpha$-MSH), $\beta$-Melanotropin ( $\beta$-MSH), und/oder $\gamma$-Melanotropin ( $\gamma$-MSH) sowie Peptid-Teilstücken dieser Substanzen als Wirkstoff zur Therapie von Neuralgien, Neutriden und rheumatischen Erkrankungen, Folgezuständen nach und infolge von Polioinfektionen, der Lateralsklerose und rheumatischen Erkrankungen und der Gicht vorgeschlagen.

Als Melanotropin bezeichnet man im Hypophysenzwischenlappen gebildete Peptidhormone, deren Sekretion durch den Hypothalamus-Faktor gehemmt wird. Bei $\alpha$-Melanotropin ( $\alpha$-MSH) handelt es sich um ein lineares Polypeptid mit dreizehn Aminosäuren, während es sich bei $\beta$-MSH (Mensch) um ein Polypeptid mit achtzehn Aminosäuren handelt. Als $\gamma$-Melanotropin ( $\gamma$-MSH) wird ein Polypeptid mit zwölf Aminosäuren bezeichnet. Die vorstehend genannten Substanzen sind bereits isoliert
bzw. synthetisch hergestellt worden und bekannt. Derartige Hormone werden auch als Farbwechselhormone des
Hyphophysenzwischenlappens bezeichnet. Es ist bekannt,
daß diese Hormone eine Erweiterung der dunklen (und
roten) Pigmentzellen bei kaltblütigen Wirbeltieren bewirken.

Es wurde nunmehr gefunden, daß sich diese Substanzen
in ausgezeichneter Weise als Wirkstoff zur Therapie der
vorstehend geschilderten Krankheitsbilder eignen.

Es sei darauf hingewiesen, daß beim synthetisch zugänglichen $\alpha$-MSH die Folge der dreizehn aufbauenden Aminosäuren der des Hormons ACTH entspricht. Die Fachwelt
war zwar bisher aufgrund von Untersuchungen mit Polypeptidfragmenten und synthetischen Polypeptiden der
Auffassung, daß für die biologische Aktivität des ACTH
nicht das gesamte Molekül benötigt wird; hierbei wurde
jedoch synthetischen Polypeptiden, die aus neunzehn
oder dreiundzwanzig Aminosäuren bestehen, die vollständige
biologische Aktivität (Corticotrophe Wirkung) zugesprochen. Man war
der Auffassung, daß zur Erzielung dieses Effektes das
Polypeptid mindestens 19 Moleküle aufweisen müsse. Die
erfindungsgemäßen Wirkstoffe basieren nicht auf diesem
corticotrophen Effekt.

Als Beispiele für die Anwendung des MSH-Wirkstoffes
seinen entzündliche rheumatische Erkrankungen, wie
beispielsweise chronische Polyathritis, Spondylarthritis
ankylopoetica,

degenerative rheumatische Erkrankungen, wie beispielsweise Arthrosen, Spondylosen, extraartikuläre rheumatische Erkrankungen, wie beispielsweise Muskelrheumatismus, und pararheumatische Erkrankungen, insbesondere Kollagenosen genannt. Der Wirkstoff ist ferner zur Therapie der Gicht (Hyperurikämie) geeignet. Er ist desweiteren zur Behandlung von Neuralgien (anfallsweise auftretenden Schmerzen in der Bahn eines sensiblen oder gemischten Nerven) oder Neuritiden (Nervenentzündungen) der verschiedenen Erscheinungsformen geeignet.

Die Wirkstoff können sowohl in reiner Form als auch als therapeutische Mittel in Verbindung mit üblichen Träger (Inert)- und Hilfsstoffen appliziert werden, wobei grundsätzlich sämtliche möglichen Applikationsformen geeignet sind. Da die Wirkstoffe ausserordentlich empfindlich sind und sehr rasch ihre Wirksamkeit verlieren, sollten sie vorzugsweise zusammen mit einer Depotsubstanz appliziert werden. Der Wirkstoff kann dabei beispielsweise an einen anorganischen Zinkkomplex adsorbiert werden.

Der Wirkstoff wirkt als $\alpha$-MSH praktisch in gleicher Weise wie als $\beta$-MSH, was darauf zurückzuführen ist, daß beide Stoffe eine identische Sequenz von sieben Aminosäuren haben, nämlich Met-Glu-His-Phe-Arg-Trp-Gly. Erfindungsgemäß kann daher auch das Polypeptid der entsprechenden Aminosäurensequenz selbst eingesetzt werden.

Die besten Ergebnisse wurden mit dem Tetrapeptid His-Phe-Arg-Trp erzielt, das im $\alpha$-, $\beta$- und $\gamma$-MSH vorkommt. Dieses Tetrapeptid His-Phe-Arg-Trp wird als eigentlich biologischer aktiver Teil des MSH angesehen.

Erfindungsgemäß wird ferner ein Wirkstoff zur Therapie der im Anspruch 3 angegebenen Krankheitsbilder vorgeschlagen.

Die mit dem erfindungsgemäßen Mittel durchgeführten Untersuchungen haben zu folgenden Ergebnissen geführt:

Es wurde anfangs versucht, durch UV-A-Strahlung die körpereigene Melanotropinbildung zu stimulieren. Dabei wurde festgestellt, daß nach ca. 15 - 30 Minuten kühler Besonnung einzelner Hautpartien den gesamten Körper sehr merklich ein Gefühl der Kräftigung und Frische durchströmte. Es ergab sich eine viel bessere Gliederbeweglichkeit. Über den gesamten mehrwöchigen Zeitraum der Behandlung hinweg war eine deutliche Zustandsverbesserung festzustellen, wobei allerdings auffällig war, daß der oben beschriebene Effekt um so länger auf sich warten ließ, je stärker vorpigmentiert der zur Besonnung ausgewählte Hautabschnitt bereits war.

Es wurden ferner die chemisch reinen Substanzen $\alpha$-MSH und $\beta$-MSH subkutan bei einer Reihe von Patienten gespritzt. Dabei trat regelmäßig bereits innerhalb der ersten Stunde ein kurzzeitiger Besserungseffekt (Kräftigung, Frischegefühl) auf, bei der erstmaligen Applikation unter Umständen auch erst nach ein bis zwei Tagen. Dabei wurde festgestellt, daß $\alpha$-MSH praktisch in gleicher Weise wirkt wie $\beta$-MSH, was offenbar auf die identische Sequenz der sieben Aminosäuren zurückzuführen ist. Schließlich wurde auch das vorstehend erwähnte Tetrapeptid allein gespritzt, wobei sich ergab, daß dieses Peptid im wesentlichen die gleiche Wirkung wie $\alpha$-MSH und $\beta$-MSH besitzt. $\alpha$-MSH ist jedoch viel aufwendiger in der Herstellung als das Tetrapeptid.

- 8 -

Weitere vergleichende Versuche mit verschiedenen Peptiden aus sechs, vier bzw. zwei Aminosäuren der Siebenerkette Met-Glu-His-Phe-Arg-Trp-Gly ließen erkennen, daß die Substanz mit der stärksten Positivwirkung und der schwächsten Negativwirkung das vorstehend erwähnte Tetrapeptid His-Phe-Arg-Trp ist.

Die Wirkung von etwa 0,3 mg $\alpha$-MSH entsprach etwa der Wirkung von 0,1 mg dieses Tetrapeptides.

Das Tetrapeptid His-Phe-Arg-Trp wurde in einem Langzeitversuch über 2 - 15 Monate hinweg einer Reihe von Patienten subkutan appliziert. Dabei wurden folgende Ergebnisse erhalten:

Zwei Drittel der Patienten hatten ein subjektives - aber durchaus auch objektivierbares - Gefühl der Frische, höheren Lebensmut und größerer Leistungsfähigkeit. Es traten keine Verschlechterungen des Krankheitsbildes auf, sondern einige nachweisbare Verbesserungen.

Bei diesen Patienten war ein starkes Nachlasen der Spastik festzustellen. Ausserdem wurde von den einzelnen Patienten berichtet: Normalisierung des (bisher zu niedrigen) Blutdrucks, bessere Durchblutung und Beweglichkeit der Extremitäten, Wiederherstellung des Tag-Nacht-Rhythmus (keine Schlafstörungen mehr), bessere Sehfähigkeit, Normalisierung des Zyklus, Minderung von Gleichgewichtsstörungen. Verschiedene Anhaltspunkte machten deutlich, daß es sich nicht um einen Cortisoneffekt handelte. Cortison und Melanotropin bzw. das Tetrapeptid His-Phe-Arg-Trp wurden gut nebeneinander

0146113

- 9 -

vertragen. Das Absetzen von Cortison wurde durch das Tetrapeptid wesentlich erleichtert und ohne Rückschläge oder Komplikationen beschleunigt.

Wenn ein bis zwei Tage lang nicht oder mit Placebos behandelt wurde, trat bei allen Patienten eine objektive Verschlechterung in Richtung der bisherigen Beschwerden auf; auch subjektiv waren alle Patienten eindrucksvoll in der Lage, anhand der Wirkung Spritzen mit Substanz von Placebos zu unterscheiden. Die richtige Dosierung des erfindungsgemäßen Mittels ist kritisch. Überdosierungen machten sich in Form von erhöhtem Blutdruck, Hitzegefühl, Kurzatmigkeit und bei kreislauflabilen Patienten in deren typischen Beschwerden bemerkbar. Die normale Tagesdosis des Tentrapeptides zur MS-Therapie wurde auf etwa 100 - 200 µg festgesetzt, in Einzelfällen - solange es ohne die obengenannten Überdosierungsphänomene vertragen wird - höher bzw. im Sinne einer Erhaltungsdosis oder bei kreislauflabilen Patienten auch niedriger.

Optimale Wirkungen sind nur bei optimalem Melanotropinspiegel erreichbar, so daß auch Überdosierungen die positive Wirkung der Substanz beeinträchtigen können.

Die geeignete Dosis scheint nicht nur individuell von Patient zu Patient unterschiedlich zu sein, sondern auch bei jedem einzelnen Patient laufend der aufmerksamen Feinjustierung bedürfen.

Die Erfahrungen mit $\alpha$-, $\beta$- und $\gamma$-MSH bzw. dem erwähnten Tetrapeptid zeigen, daß diese Substanzen ausserordentlich empfindlich und labil sind.

- 10 -

Beispielsweise ist das Tetrapeptid zwar als Reinsubstanz tiefgekühlt lagerfähig; in destilliertem Wasser gelöst und bei Kühlschranktemperatur aufbewahrt verliert es jedoch pro Woche etwa die Hälfte seiner Wirksamkeit. Im Körper büßt der Stoff innerhalb kurzer Zeit seine Wirkung ein, so daß sich etwa folgende Dosierung als zweckmäßig erwies: Alle 2 - 3 Stunden 10 - 30 µg des Tetrapeptides in 0,4 ml destilliertem Wasser subkutan. Bei der Verbindung des Tetrapeptides mit einer Depotsubstanz sollte eine Wirkungsdauer von 12-15 Stunden angestrebt werden.

Es wurde ferner festgestellt, daß die erfindungsgemäßen Substanzen eine Verbesserung der Sehfähigkeit des menschlichen Auges bewirken. Diese Fähigkeit zeigte insbesondere das Tetrapeptid His-Phe-Arg-Trp. Die Fähigkeit des Patienten in bezug auf die Zusammensetzung von bewegten Bildern wurde durch Verabreichung des Tetrapeptides in der Form von Augentropfen wesentlich verbessert; auch die Wahrnehmung von Lichtreizen konnte stabilisiert werden.

Es wurden täglich ca. 2µg pro Auge des Tetrapeptides in physiologischer Kochsalzlösung in der Form von Augentropfen verabreicht. Dabei wurden die vorstehend erwähnten Erfolge erzielt.

Um die therapeutische Wirksamkeit des erfindungsgemäßen Mittels zu erhöhen, wurde der Wirkstoff mit einer Depotsubstanz kombiniert.Als Depotsubstanz wurde Aluminiumhydroxid (Al $(OH)_3$) verwendet, das beispiels-weise von der Firma SERVA unter der Bezeichnung Alu-Gel-S pharm. vertrieben wird und bei dem es sich um eine 2%-ige Suspension handelt.

- 12 -

Ein derartiges Depotpräparat wurde in der folgenden
Weise hergestellt:

Das Tetrapeptid wurde in Äthanol aufgelöst, wobei pro
mg Tetrapeptid 0,2 ml Äthanol verwendet wurden. Es
wurden pro mg Tetrapeptid 0,2 - 0,3 ml des o.a. Aluminiumhydroxides zugesetzt. Das entstandene Präparat
ließ man unter Schütteln etwa 10 Minuten reagieren.
Danach wurden 5 ml destilliertes Wasser zugesetzt.

Während die Halbwertzeit des reinen Tetrapeptides ca.
1 Woche beträgt, trat bei dem hergestellten Depotpräparat ein geringerer Wirkungsverlust als 10% pro
Woche auf. Im Körper hält der Depoteffekt zwischen
6 - 12h an. Als Tagesdosis von dem Depotpräparat
empfiehlt sich eine Menge von etwa 1 ml, was etwa 200
Mikrogramm entspricht.

Das erfindungsgemäße Mittel eignet sich mit oder ohne
Depotsubstanz besonders gut zur perkutanen Applikation.
Um eine entsprechende Applikationsform (Salbe) herzustellen, wurde der Wirkstoff bzw. das vorstehend beschriebene Depotpräparat mit einem Öl pflanzlicher Herkunft vermischt. Als Öl wurde dabei eine Substanz mit dem
Handelsnamen Miglyol 812 von der Firma Dynamit-Nobel
verwendet. Dieses Präparat hat insbesondere bei Frauen
zu guten therapeutischen Erfolgen geführt. Zur therapeutischen Behandlung von Männern wird als perkutane
Applikationsform anstelle des Zusatzes von Öl der Zusatz von Wasser vorgeschlagen. Hiermit lassen sich bessere
Ergebnisse erreichen.

Das in der vorstehend beschriebenen Weise hergestellte Depotpräparat wurde über 3 Monate zehn Patienten verabreicht. Es wurde dabei etwa die gleiche Erfolgsquote erzielt wie bei den vorstehend beschriebenen Untersuchungen, wobei jedoch insgesamt ein besserer therapeutischer Heileffekt erzielt wurde als bei einer stoßweisen Applikation.

Für das Depotpräparat ist keine gekühlte Lagerung mehr erforderlich. Das Präparat kann bei normaler Zimmertemperatur gelagert werden.

Das genannte Depotpräparat kann ebenfalls in der Form von Augentropfen appliziert werden. Die Augentropfen werden durch Verdünnen des Depotpräparates mit physiologischer Kochsalzlösung hergestellt.

Die Parallelwirkung des erfindungsgemäßen Wirkstoffs in bezug auf Nervenkrankheiten und Hautkrankheiten wird darauf zurückgeführt, daß Nervenzellen und Hautzellen eine gemeinsame neonatale bzw. embryonale Vorform besitzen, die als Melanoblasten bezeichnet wird.

Die vorliegende Erfindung betrifft desweiteren einen Wirkstoff bzw. ein therapeutisches Mittel zur Behandlung von Zuständen infolge überhöhten Melanotropinspiegels, insbesondere hormonal bedingten Bluthochdrucks.

Als Melanotropin bezeichnet man im Hypophysenzwischenlappen gebildete Peptidhormone. Nach einem Vorschlag des Erfinders werden derartige Substanzen zur Behandlung von Fehlfunktionen des menschlichen Diencephalon und/oder der Hypophyse und zur Behandlung von Neuralgien, Neuritiden, rheumatischen Erkrankungen etc., eingesetzt.

Werden diese Substanzen in Überdosierungen verwendet,
können unter Umständen geringfügige Beschwerden auftreten, die sich beispielsweise in einem erhöhten
Blutdruck äußern.

Der Erfindung liegt die Aufgabe zugrunde, einen Wirkstoff
bzw. ein therapeutisches Mittel zur Behandlung von Zuständen infolge überhöhten Melanotropinspiegels, insbesondere hormonal bedingten Bluthochdrucks, zur Verfügung zu stellen.

Diese Aufgabe wird erfindungsgemäß durch einen Wirkstoff
mit den Merkmalen des Patentanspruchs 14 und ein therapeutisches Mittel mit den Merkmalen des Patentanspruchs
15 gelöst.

Durch Untersuchungen wurde festgestellt, daß die vorstehend genannten Substanzen in der Lage sind, den
Melanotropinspiegel zu senken. Insbesondere wurde festgestellt, daß durch Verabreichen dieser Substanzen eine
Behandlung hormonal bedingten Bluthochdrucks möglich
ist, der sich beträchtlich reduzieren läßt. Das erfindungsgemäße Mittel kann somit als Korrektiv eingesetzt werden,
beispielsweise bei versehentlicher Überdosierung von
Melanotropin bzw. seinen Peptiden. Ferner kann das erfindungsgemäße Mittel als eigenes Medikament zur Normalisierung hormonal bedingten Bluthochdrucks verwendet
werden.

Besonders gute Ergebnisse sind mit dem Mittel erreicht
worden, das aus einem Gehalt des Hormons MIF (MSH-
Release inhibiting factor) besteht. Dieses Hormon wird
im Hypothalamus erzeugt und entspricht der Substanz
Prolyl-Leucylglycinamid (Pro-Leu-Gly-NH2). Die Substanz
ist als solche bekannt und im Handel erhältlich.

- 14 -

Die Erfindung bezieht sich ferner auf eine Substanz, die aus Teilstücken eines derartigen Polypeptides besteht, beispielsweise aus einem Polypeptid mit der Aminosäurensequenz Leu-Gly-NH$_2$.

Für das erfindungsgemäße Mittel sind grundsätzlich alle Applikationsformen geeignet. Durch Verabreichung des Mittels in einer parenteralen Applikationsform wurden gute Ergebnisse erzielt.

Die vorstehend aufgeführten Ergebnisse wurden durch Untersuchungen bestätigt. Das Mittel wurde an eine Reihe von Patienten, die an hormonal bedingtem Bluthochdruck leiden, parenteral verabreicht, wobei pro Tag eine Dosis von 10 - 30 µg gegeben wurde. Durch Verabreichung des erfindungsgemäßen Mittels konnte eine beträchtliche Reduzierung des Blutdruckes von Werten über 200 bis in normale Bereiche hinein erreicht werden.

Weitere Untersuchungen zeigten, daß das Mittel ebenfalls als Korrektiv bei Verabreichungen von zu hohen Melanotropinmengen verwendet werden kann. Die durch derartige Überdosen erzeugten Beschwerden (beispielsweise erhöhter Blutdruck) konnten durch Verabreichung des Mittels behoben werden.

Das Mittel wurde ferner oral (über die Mundschleimhäute) in Tropfenform appliziert. Dabei kamen Mengen von 100 bis 200 µg pro Tag zur Anwendung. Die vorstehend aufgeführten Ergebnisse wurden bestätigt.

Die Erfindung schlägt ferner Kombinationspräparate aus den Wirkstoffen der Patentansprüche 1 bis 3 sowie dem Wirkstoff aus dem Patentanspruch 14 vor. Durch geeignete Abstimmung der jeweiligen Wirkstoffe lassen sich therapeutische Mittel konzipieren, die eine gute
therapeutische Wirkung entfalten, ohne dabei die
vorstehend geschilderten Nachteile aufzuweisen.

Patentansprüche:

1. α-Melanotropin ( α-MSH), β-Melanotropin ( β-MSH) und/oder ϰ-Melanotropin ( ϰ-MSH) sowie Peptid-Teil-stücke dieser Substanzen zur Anwendung als Wirkstoff zur Therapie von Fehlfunktionen des menschlichen Diencephalon und/oder der Hypophyse und dadurch bedingter Störungen, beispielsweise des Blutdrucks, der Blasenfunktion, des hormonalen Gleichgewichts, des Stoffwechsels, der Keimdrüsenfunktion, des Wachstums, der Wärmeregulation, des Wasserhaushalts, des Nervensystems und des psychischen Gleichgewichts, insbesondere zur Behandlung der Multiplen Sklerose.

2. α-Melanotropin ( α-MSH), β-Melanotropin ( β-MSH) und/oder ϰ-Melanotropin ( ϰ-MSH) sowie Peptid-Teil-stücke dieser Substanzen zur Anwendung als Wirkstoff zur Therapie von Neuralgien, Neuritiden, rheumatischer Erkrankungen, Folgezuständen nach und infolge von Polioinfektionen, der Gicht und der Lateralsklerose, insbesondere zur Therapie entzündlicher rheumatischer Erkrankungen, degenerativer rheumatischer Erkrankungen, extraartikulärer rheumatischer Erkrankungen und/oder pararheumatischer Erkrankungen.

3. α-Melanotropin ( α-MSH), β-Melanotropin ( β-MSH) und/oder ϰ-Melanotropin ( ϰ-MSH) sowie Peptid-Teil-stücke dieser Substanzen zur Anwendung als Wirkstoff zur Therapie von Hautkrankheiten, Erkrankungen der Schleimhäute sowie Allergien, insbesondere von Herpes, Schuppenflechte, Ekzemen und Heuschnupfen.

4. Wirkstoff nach einem der vorangehenden Ansprüche, dadurch gekennzeichnet, daß er aus einem Polypeptid der Aminosäurensequenz Met-Glu-His-Phe-Arg-Trp-Gly besteht.

5. Wirkstoff nach einem der Ansprüche 1 bis 3, dadurch gekennzeichnet, daß er aus einem Polypeptid der Aminosäurensequenz Met-Gly-His-Phe-Arg-Trp besteht.

6. Wirkstoff nach einem der Ansprüche 1 bis 3, dadurch gekennzeichnet, daß er aus dem Tetrapeptid His-Phe-Arg-Trp besteht.

7. Wirkstoff nach einem der Ansprüche 1 bis 3, dadurch gekennzeichnet, daß er aus $\beta$-MSH der verschiedenen Varianten(Tier-Mensch) besteht.

8. Wirkstoff nach einem der Ansprüche 1 bis 3, dadurch gekennzeichnet, daß er aus den Peptid-Teilstücken His-Phe, Phe-Arg oder Arg-Trp besteht.

9. Wirkstoff nach einem der Ansprüche 1 bis 3, dadurch gekennzeichnet, daß er aus einem Polypeptid der Aminosäurensequenz Met-Glu-His-Phe-Arg-Trp besteht.

10. Wirkstoff nach einem der Ansprüche 1 bis 3, dadurch gekennzeichnet, daß er aus einem Polypeptid der Aminosäurensequenz (H-Met $(O_2)$-Glu-His-Phe-D-Lys-Phe-OH besteht.

11. Wirkstoff nach einem der Ansprüche 1 bis 3, dadurch gekennzeichnet, daß er aus einem Polypeptid der Aminosäurensequenz H-His-Phe-Arg-Trp-OH • 2AcOH besteht.

12. Therapeutisches Mittel enthaltend einen Wirkstoff nach einem der Ansprüche 1 bis 11.

13. Mittel nach Anspruch 12, dadurch gekennzeichnet, daß es als Injektionslösung parenteral,in Form einer Salbe perkutan, als Augentropfen oder über die Schleimhäute von Nase und Mund applizierbar ist.

14. Mittel nach einem der Ansprüche 11 bis 13, dadurch gekennzeichnet, daß es eine Depotsubstanz, insbesondere Aluminiumhydroxid, umfaßt.

15. Polypeptid mit der Aminosäurensequenz Pro-Leu-Gly-$NH_2$ bzw. Teilstücken davon, insbesondere Pro-Leu, PZ-Pro-Leu, DNP-Pro-Leu-Gly, Pro-Leu-Gly-$NH_2$ · $1/2H_2O$, Z-Pro-Leu-Gly-$N_2$, DNP-Pro-Leu-Gly-Ile-Ala-Gly-Arg-$NH_2$ oder PZ-Pro-Leu-Gly-Pro-D-Arg · $2H_2O$ zur Anwendung als Wirkstoff zur Behandlung von Zuständen infolge überhöhten Melanotropinspiegels, insbesondere hormonal bedingten Bluthochdrucks.

16. Therapeutisches Mittel enthaltend einen Wirkstoff nach Anspruch 15.

17. Mittel nach Anspruch 16, dadurch gekennzeichnet, daß es alsInjektionslösung parenteral, in Form einer Salbe perkutan, als Augentropfen oder über die Schleimhäute von Nase und Mund applizierbar ist.

18. Mittel nach Anspruch 16 oder 17, dadurch gekennzeichnet, daß es eine Depotsubstanz, insbesondere Aluminiumhydroxid, umfaßt.

0146113

- 4 -

19. Therapeutisches Mittel enthaltend einen Wirkstoff
nach einem der Ansprüche 1 bis 11 und einen Wirkstoff
nach Anspruch 15.

20. Wirkstoff nach einem der Ansprüche 1 bis 3, dadurch
gekennzeichnet, daß er aus einem Polypeptid der Aminosäurensequenz H-His-Phe-Arg-Trp-OH x HCL besteht.